# EUROPEAN PATENT APPLICATION

(11) **EP 4 768 048 A2**
(43) Date of publication of application: **01.07.2026**
(21) Application number: 25227847.8
(22) Date of filing: 31.12.2025
(51) Int. Cl.: A61L 9/20, F24F 8/22

(54) **INDOOR AIR QUALITY ENHANCEMENT UVC EXCHANGER AND MULTIFILTRATION**

(30) Priority: 30.12.2024 PT 2024119966
(71) Applicant: Oprimee - Innovation Design Engineering Solutions, Lda, 3405-155 Oliveira do Hospital (PT)
(72) Inventor: DOS SANTOS ALMEIDA NUNES, JOÃO MIGUEL, 3405-155 OLIVEIRA DO HOSPITAL (PT)
(74) Representative: Patentree

(57) **Abstract**

The present disclosure relates to an air exchanger device for improving indoor air quality in confined or semi-confined spaces. The device comprises at least one air intake area and one air exit area connected by a sinuous circuit, where air or gas flows and is exposed to at least one UV-C radiation lamp, particularly far-UV-C, for inactivating microorganisms. The method of operation involves drawing air or gas through the intake area, directing it through the sinuous circuit, exposing it to UV-C radiation, and releasing the treated air through the exit area. The geometry and effective length of the sinuous circuit are selected to control airflow velocity and residence time such that, for a predetermined airflow rate, a minimum effective UV-C dose is delivered to the air flowing through the device. Applications of the device include improving air quality in building ventilation systems and transportation systems. Additional features are disclosed, including detachable cartridges that can be configured as activated carbon filters, radon filters, water filters, or combinations thereof, to further enhance air quality, as well as real-time air quality monitoring through a control unit for improved performance and user convenience.

## Description

### TECHNICAL FIELD

The present disclosure relates to air quality improvement systems, specifically to devices and systems designed to improve indoor air quality in confined or semi-confined spaces occupied by people or animals. The disclosure addresses air purification through UV-C radiation and filtration technologies, while integrating advanced monitoring, control, and communication functionalities for enhanced performance and user convenience.

### BACKGROUND

Concern about the presence of microorganisms and chemical pollutants in the air, as well as their harmful effects on health, has been increasing on a large scale. There were always microorganisms in the environment, however some of them are pathogenic, reason why they cannot live in symbiosis with other living organisms. Moreover, when the presence of microorganisms which live in symbiosis in the environment is higher than expected, they can also become pathogenic, making it a public health problem. The problem is even worsened in confined and semi-confined spaces used by many people and or animals.

The current state of the art faces a significant challenge in balancing energy efficiency with thermal comfort and indoor air quality, which often appear to be at odds in existing solutions. This issue arises due to the introduction of fresh air exchanges that, while essential for ventilation, frequently result in the increased presence of harmful physical particles, particularly microorganisms. Additionally, improper airflow dynamics exacerbate the problem, leading to suboptimal air distribution and further compromising indoor air quality and comfort. Addressing this dual challenge requires innovative approaches that simultaneously optimize energy consumption, ensure adequate thermal comfort, and maintain high standards of indoor air quality.

Several proposals for air purification to minimize harmful effects of microorganism presence have emerged, especially in confined and semi-confined spaces used by people. Due to their wide diversity, their specific resistant capacities are different, which leads to extreme procedures of microorganism inactivation, such as for example, inactivation through exposure to pH <2 and exposure to temperatures above 65ºC for long periods of time. However, they may both be harmful for human safety, wellness and comfort.

For example, microorganism inactivation by exposure to low pH, besides the high costs needed, originates harmful volatile compounds. Inactivation by exposure to high temperatures, besides representing substantial economic and energy costs, may be limited due to the existence of thermally stable microorganisms, which require really high temperatures for a long period of time for its inactivation.

On contrary, Ultraviolet (UV) radiation presents itself as an excellent alternative, given its relatively low cost and its potential effectiveness in microorganisms inactivation (for thousand years, no viruses or bacteria are known to have withstood UV radiation). UV radiation as a disinfection agent must be planned carefully since the inhibition of microbiologic activity highly depends on the type of microorganism and its resistance to radiation. Moreover, there are physio-chemical factors that influence technology efficiency, such as heat and mass transmission phenomena, the relative humidity, the loss of charges in the system and the distance between the radiation and the target. These factors are considered in the development of the present disclosure to maximize its effectiveness, as well as to ensure the user's safety.

Exposure time calculus and activity decay of different microorganisms when subjected to UV radiation of different wavelengths and intensities monitoring are already reported and scientifically described in literature. UV radiation covers a range of wavelengths from electromagnetic spectrum (100 nm to 400 nm), but it is in the range of ultraviolet-C (UV-C) rays (~200 nm - 280 nm, peak at ~254 nm) that germicidal wavelength falls within, which is effective for microbiological activity inactivation, but also has high cytotoxicity, carcinogenic and mutagenic activity for human cells, reason why it raises concerns about the people's level of exposure.

However, according to biophysical principles, lower wavelengths (~200 nm) are cytotoxic and mutagenic to microorganisms, but not to human cells. This phenomenon can be explained by the fact that UV radiation in this range of wavelengths is absorbed by proteins and other biomolecules, so its ability to penetrate biological material is very limited.

The key skin cells at risk from UV exposure, such as basal cells and melanocytes, are located within the epidermis below the stratum corneum which is a 5 to 20 mm thick layer of dead non-nucleated cells. Given its very limited penetration (half-value thickness 0.3 mm), approximately 200 nm light will thus have minimal ability to penetrate the stratum corneum and reach the underlying key skin cells such as basal cells or melanocytes. Moreover, UV-C light between 207 nm and 227 nm cannot penetrate either the ocular tear layer (and nor even the cytoplasm of individual human cells), reason why it does not represent a threat for human eyes, in contrast to the conventional germicidal UV light which can reach these sensitive cells.

Furthermore, at the cellular level, bacteria are much smaller than almost any human cell. Typical bacterial cells are less than 1 µm in diameter, whereas typical human cells diameter ranges from about 10 to 25 µm. It follows that ~200 nm UV light can penetrate throughout typical bacteria but cannot penetrate significantly beyond the outer perimeter of the cytoplasm of typical human cells and will be drastically attenuated before reaching the human cell nucleus.

The verification of this information has already been scientifically reported and it has been described that UV-C radiation between 207 nm and 222 nm demonstrated microbiological inactivation capacity without showing significant cytotoxicity for human cells.

In the last few years, as a result of the increasing ease with which information is disseminated, many people have begun to expose air quality related problems. Outbreaks of transmissible diseases by pathogenic microorganism inhalation and, in particular the recent Covid-19 pandemic, have increased the need of "air purification" solutions in confined and semi-confined spaces. Moreover, comfort needs are also growing, which translates into growing needs for heating or cooling confined and semi-confined spaces.

Devices such as air conditioners, fan coil units, radiators, among others, are among the equipment that are commonly used due to their efficiency and versatility (given that they enable both space heating and cooling), solutions such as air conditioning or fan coil units are among those preferred by users. Fan coils are intelligent climate control systems that use a fan to distribute hot or cold air through a heat exchange coil and which can be regulated by a thermostat. Their performance is similar to air conditioners, but instead of using refrigerant gas for air conditioning, they use cold or hot water circulation. Generically, this is a device made up of a fan, a heat exchanger and a filter and are usually connected to water temperature regulation solutions such as air-water heat pumps, chillers, or boilers.

Indoor air quality systems using water face challenges with dissolved CO₂, which forms carbonic acid and lowers water pH. This can corrode components, reduce purification efficiency, and reintroduce CO₂ into the air, undermining performance. Effective solutions are needed to address these issues and enhance system reliability.

One of the most important components of this system is the heat exchanger since it is where thermal exchanges occur. Heat exchangers already have a long and varied history of use, which has led them to be constantly improved, originating several types such as multi-tube heat exchangers, concentric tube heat exchangers, plate heat exchangers, or shell and tube heat exchangers. Most of these exchangers have the sinuosity of the path that the liquid or gaseous fluid that circulates in it is forced to follow, which aims to increase the exposure time of the circulated fluid to another medium of different temperature and to increase the contact area between surfaces of different temperatures.

Moreover, a diverse range of heaters is available nowadays, each tailored to different requirements and environments. Electric heaters, such as convectors and panel heaters, provide swift solutions for smaller spaces, while halogen heaters utilize infrared lamps for heat generation. Natural gas heaters prove efficient for warming homes and commercial spaces, and gas water heaters are designed to meet domestic hot water needs. Oil heaters, such as radiators, deliver a gradual and sustained warmth. Ceramic heaters stand out for their effective and efficient heat distribution. Wall-mounted infrared heaters offer a direct heating approach.

The right heater depends on factors such as space characteristics, available energy sources, and individual preferences. Considerations like size area, energy efficiency, and desired heating methods also play a crucial role in selecting the most suitable model for each specific need. While heaters contribute to a comfortable temperature, they do not actively purify the air. So, it is important to integrate air renewal solutions to promote both comfort and optimal air quality.

It is also important to note that ventilated air needs to be of reasonable quality, so another particularly important component of fan coil units is their filter. By one side, the filtering system adopted guarantees protection to the system itself, as it prevents the passage of dirt and appreciable dimensions particles, potentially dangerous for the internal mechanical parts of the device. On the other side, it is through the filters that it is ensured that the air vented to the space outside the appliance has a minimum of quality and healthiness.

Filters such as HEPA filters (with good retention capacity for microparticles and microorganisms, such as viruses and bacteria) or activated carbon filters (with high retention capacity for gas particles that cause odours in the environment) are normally used. Due to grown concerns about air quality in confined and semi-confined spaces, the market began to present solutions with different degrees of innovation. Many of these solutions are adaptations of previously existing technologies, in which means for eliminating microorganisms are questionable. As follows, many air purifiers available on the market operate similarly to fans, in which the "air purification" function relies on the use of particle retention filters, such as already mentioned HEPA filters.

In addition, even if their retention capacity is high, if filters change does not occur frequently and if there is no prevention of microbiological activity, the filter will not have the desired effect. When it comes to bacteria, those filters can even have the opposite effect, due to optimal conditions for bacteria growth in the filter itself, such as temperature and humidity. Moreover, bacteria that may eventually be released when the air is expelled from the equipment. This often occurs in systems such as air conditioners, purifiers and other systems involving air circulation with particle filtration.

Furthermore, HEPA filters do not remove odours, chemicals, and gases, for that effect HEPA air purifiers have some level of activated carbon-based material to absorb odours and chemicals. They trap odours through a process called adsorption, which occurs when molecules attach to the outside of a surface, rather than being soaked into it. The more porous the activated carbon, the better, as this will increase the amount of surface space available for contaminants to latch onto when air passes through the filter. However, if care is not taken, it can become saturated, no longer being able to absorb said odours, chemicals, and gases.

There is yet another type of filter, namely electrostatic filters that have a high filtration capacity of ultrafine dust, although they can often operate with higher air speed. They remove dust from air by imposing an electric charge on the dust, in an ionizer, and then passing the suspension between some vertically hanging charged plates (electrodes). The ionized dust particle then moves towards the oppositely charged plate to which they adhere, considering its inertia, density, and charge. The plates are sometimes coated with oil to help dust retention.

Innovative solutions are already commercialized. LG, for example, produces air conditioners with microorganism inactivation technology based on Ultra-Violet (UV) micro-lamps. Their LG Dual Inverter Voice UV Nano system consists of an air conditioner, made up of three essential mechanical components: compressor, condenser, and evaporator, which work with a refrigerant gas (R-22 gas). In addition to these components, this device has the so-called UV micro-lamps inside, whose action is responsible for breaking the microorganisms DNA present in the air and for their consequent inactivation. This device uses UV-C lamps with a radiation peak at 254 nm and as mentioned above, this is a wavelength that can be very harmful to human health. Alternatively, innovations can be developed with a UV-C lamp with radiation at a wavelength between 207 nm and 222 nm, as it is not cytotoxic for the deeper layers of human skin.

Another company, Field Controls, also has an air purification system for residential environments, which is called Duo 2000. This system consists of a device that has a powerful high-intensity UVC germicidal lamp flanked by a set of filters where reactive oxidation processes occur. However, the radiation used by this equipment has warnings for human health, and it is even recommended in its "safety considerations" not to look directly at the lamps used in this equipment, which would not be needed if it was a UV lamp with emission spectrum between 207 nm and 222 nm.

In addition, there is also a technology developed by AiroDoctor, AiroDoctor V10, which is an air purification system for large indoor spaces, using an active ventilation system with HEPA filters, UV-A photocatalysis and heat recovery. As already mentioned, HEPA filters do not represent the best solution for microorganisms inactivating. Moreover, the radiation used is in the UV-A range, which is not lethal for all microorganisms, reason why lower wavelengths radiation is required.

Radioactivity is a natural process where unstable atoms release particles or radiation in order to attain stability. Radon, a radioactive gas, is commonly found in nature, particularly in areas with soil rich in uranium. Inhalation of radon poses health risks due to its radioactive decay, which can harm human tissues. To address this risk, specific filters, like radon filters, are employed. These filters are designed to capture radon particles, preventing their release into the air and minimizing exposure. Installing these filters not only enhances air quality but also reduces the potential health hazards associated with radon presence in enclosed spaces.

Moreover, it is important to mention that dehumidifiers play a crucial role in maintaining the proper humidity balance within indoor spaces. Its significance becomes apparent in effectively managing excessive moisture levels, thereby preventing issues such as mould, mildew, and the proliferation of dust mites. Beyond mere discomfort, overly humid air can lead to damage to furniture, clothing, and adverse effects on respiratory health. Operating by removing surplus moisture, the dehumidifier not only curtails mould growth but also enhances air quality, fostering a healthier and more enjoyable indoor environment. The controlled management of humidity not only safeguards the integrity of belongings but also contributes to an overall healthier and more comfortable living space.

However, regardless of the currently "air purification" system available, some disadvantages that dramatically influence the number and type of microorganisms can be found. By one side, the filters used do not retain all pathogenic microorganisms or, if they do, they do not have capacity to inactivate them, or they may even inadvertently become an "incubator" for these microorganisms. On the other side, some of the UV technologies solutions still lack the guarantee that the masses of air to be purified are subjected to sufficient exposure time to UV radiation that allows microorganisms inactivation efficiently.

In fact, existing solutions have difficulties in inactivating pathogenic microorganisms across the entire microbiological spectrum. Whereby, the intention behind the air quality enhancement system described below is to solve and overcome all issues and problems found in the current state of art.

These facts are disclosed in order to illustrate the technical problem addressed by the present disclosure.

### GENERAL DESCRIPTION

The present disclosure describes an indoor air quality enhancement exchanger and filtration equipment solution, for use in confined or semi-confined spaces occupied by people or animals, wherein it comprises at least one air intake area for extraction and at least one air exit area for insufflation. The equipment will promote the improvement of the indoor air quality by a circulation air solution with the capacity to be applied for killing and/or removing microorganisms and other physical particles that harm people's health and cause unpleasant odours and the fixation of CO₂ present in the air, without the necessity of fresh air from the outside of the space because the new fresh air from the outside decreases the energy efficiency of the buildings and space and thermal comfort.

The objective technical problem addressed by the present disclosure is to provide an indoor air treatment device that ensures reliable microbiological inactivation by UV-C radiation while avoiding insufficient exposure time and nonuniform dose distribution commonly encountered in known UV air purification systems.

The geometry and effective length of the sinuous circuit are selected to control airflow velocity and residence time such that, for a predetermined airflow rate, a minimum effective UV-C dose is delivered to the air flowing through the device.

The present disclosure is related with an air exchanger device, for improving indoor air quality in confined and semi-confined spaces, comprising: at least one air intake area (4) for air and/or gas inflow; at least one air exit area (5) for air and/or gas outflow; an internal air flow circuit (6) extending from the air intake area to the air exit area, the circuit comprising a plurality of sheets offset from one another along a direction extending from the air intake to the air exit to define a sinuous airflow path; at least one UV-C radiation source (7, 14) for emitting a UV-C radiation, arranged in the airflow circuit along the sinuous airflow path as to define a UV-C exposure zone for inactivating microorganisms present in the air or gas flow; wherein the sinuous airflow path comprises an effective length and cross-section selected such that, for a predetermined airflow rate, a residence time of the air within the UV-C exposure zone (6), in combination with an average UV-C irradiance provided by the at least one UV-C radiation source, results in a minimum effective UV-C dose greater than 10 mJ/cm² to the air flowing through the circuit; and wherein the plurality of sheets comprise a UV-reflective coating (10) having a UV-C reflectance coefficient of at least 0.70.

In an embodiment of the present disclosure, the air quality enhancement exchanger and filtration used in indoor confined and semi-confined spaces, for example buildings, comprises a connection between the at least one air intake area for extraction and the at least one air exit area for insufflation in the equipment that will promote the correct mechanical fluids air dynamics of the confined and semi-confined spaces occupied by people or animals.

In an embodiment of the present disclosure, the air quality enhancement exchanger used in indoor confined or semi-confined spaces comprises one controlled force air inflow action system that may be located on the up of the equipment to promote the maximum efficiency of the circulation air in the confined and semi-confined spaces occupied by people or animals, to prevent the death layer of confined and semi-confined spaces occupied by people or animals, because the natural dynamics of the air in the confined and semi-confined spaces occupied by people or animals is from bottom to top, originated by the direction of the heat flow in indoor confined or semi-confined spaces.

In an embodiment, the air quality enhancement exchanger and filtration used in indoor confined or semi-confined spaces comprises at least one controlled force air outflow action system installed in the equipment to promote the up-down air fluxes in confined or semi-confined spaces.

In an embodiment, the air quality enhancement exchanger and filtration used in indoor confined or semi-confined spaces comprises at least one controlled force air outflow action system which will enable work with (1) one upper forced air flow equipment; or (2) bottom forced air flow equipment; or (3) one upper forced air flow equipment and a bottom forced air flow equipment, take into account the power need to (1) win the loss of load in the air flux through the equipment, while also allowing a precise control of the air velocity inside the equipment, which is very important for guaranteeing the quality and efficiency of the UVC's performance, (2) and the dimension of the spaces that will applied the equipment.

In an embodiment, the air quality enhancement exchanger and filtration used in indoor confined or semi-confined spaces occupied by people or animals comprises a connection between the at least one air intake area for extraction, the at least one air exit area for insufflation with an inner sinuous circuit to apply the UVC radiation at the physical particles present in the air to ensure the exposition time and decay.

In an embodiment, the at least one controlled force air inflow action system at the bottom of the equipment for air and/or gas inflows, and the at least one controlled force air outflow action system at the bottom of the equipment for air and/or gas outflows comprise an integrated information collection, command, and control system, that integrates a CO2, CO and O2 sensors to analyse the CO₂ and O2 present in the indoor confined and semi-confined spaces, and when CO₂ or CO increases to a concentration level or O2 decreases to concentration levels that indicate a loss of air quality the solution has a visual alarm, by a light, a sonorous alarm, and a Bluetooth, Wifi or SCard sinal emissions system to inform the person or the operator in charge of the air quality therein.

In an embodiment, the air quality enhancement exchanger and filtration used in indoor confined and semi-confined comprises a system able to present data related to the "purified" air in real time, through a dashboard, to improve interaction between users and confined and semi-confined spaces.

In an embodiment, the air quality enhancement exchanger and filtration for indoor confined and semi-confined spaces comprises a system equipped with sensors available for data collecting.

In an embodiment, the air quality enhancement exchanger and filtration for indoor confined and semi-confined spaces comprises a system equipped with sensors available for emitting audible alarms when certain configurations exceed certain predefined limits.

In an embodiment, the air quality enhancement exchanger and filtration for indoor confined and semi-confined spaces comprises an adjustable size according to the implementation area.

In an embodiment, the method of functioning for an air quality enhancement exchanger and filtration, in which motors can be fitted so that it becomes an independent piece of equipment, thus not being coupled to other equipment previously installed in confined or semi-confined spaces occupied by people or animals.

In an embodiment, the air quality enhancement exchanger and filtration used indoor confined or semi-confined spaces is wherein the at least one controlled force air inflow action system and/ or the at least on controlled force air outflow action system comprise at least one flow regulator valve, cut-off valves, mechanical and electrical actuation systems for blowers/ fans and/ or rotors and/ or turbines.

In an embodiment, the air quality enhancement exchanger and filtration used in indoor confined or semi-confined spaces comprises a sinuous circuit designed to increase the time the air and/or gas stays inside the equipment and, consequently, the time of exposure to UVC radiation and decay for inactivation that will ensure the air purification.

In an embodiment, the air quality enhancement exchanger and filtration for use in confined or semi-confined spaces comprises a system able to percolate the air mass in the treatment along a circuit permanently exposed to UVC radiation with such characteristics that increase the time air and/gas and physical particles stays inside the equipment.

In an embodiment, the air quality enhancement exchanger and filtration for use in confined or semi-confined spaces comprises UVC radiation with such length that allows the maximization of the exposure time of the percolated air to radiation to effectively inactivate any microorganism.

In an embodiment, the free area of the sinuous circuit through which the contaminated air passes must be equal to or less than the inlet area of the air to be "purified".

In an embodiment, the air quality enhancement exchanger for indoor confined and semi-confined spaces comprises at least one removable far UVC lamp with radiation emission with wavelength between 190 nm and 250 nm and/or UVC lamp 253 nm and 255 nm.

In an embodiment, the air quality enhancement exchanger for indoor confined and semi-confined spaces comprises an individual detection system from the foundry of the at least one removable far UVC lamp with radiation emission between 190 nm and 250 nm and/ or UVC lamp 253 nm and 255 nm.

In an embodiment, the air quality enhancement exchanger for indoor confined and semi-confined spaces comprises at least one UV-C lamp, according to the size of the equipment.

In an embodiment, the air quality enhancement exchanger contains cartridges that can be added, collectively or individually, according to the equipment user's needs.

In an embodiment, the air quality enhancement exchanger cartridges that can be added collectively or individually comprise at least one air intake area, at least one air exit area and at least one small compartment at the bottom of the cartridge.

In an embodiment, the cartridges comprise a system that extracts air from the indoor space and directs it through a water filtration system equipped with a UVC or far-UVC lamp to inactivate microorganisms present in the water. This ensures the safety of the cartridge and its water, which can then be safely discharged into internal or external sewage systems without posing risks to human or public health. The system also improves efficiency by capturing a significant portion of particles in the initial filtration stage, thereby reducing particle deposition on the surfaces of UVC or far-UVC emission systems, such as lamps or similar devices. This minimizes efficiency losses, maintenance issues, and associated costs.

In an embodiment, the air quality enhancement exchanger small compartment at the bottom of the cartridge that can be added collectively or individually comprises at least one removable far UV-C lamp with radiation emission with wavelengths between 190 nm and 250 nm and/ or UVC lamp 253 nm and 255 nm.

In an embodiment, the air quality enhancement exchanger for indoor confined or semi-confined comprises an improvement of its physical and chemical dimensions with a cartridges system of protective filters, apart from improving its microbiological dimensions.

In an embodiment, the cartridges comprise at least one removable filter on each air and/or gas inlet or outlet, outside of the equipment.

In an embodiment, the cartridges comprise a filter system to improve odour quality and minimize the presence of small, micro and nano dimension particles, such as pollen, dust, dust mite, textile and non-textile fibres, among others types of physical particles that would contribute for health problems.

In an embodiment, the cartridges comprise a filter with smaller-sized pores and high surface contact area, such us, active carbon, capable of filtering smaller, micro and nano particles and radioactive molecules, like radon gas.

In an embodiment, the cartridges comprise a heat source for increasing contact temperature of the air and/or gas the inflows and outflows. This heat source may present a range of temperatures between 50ºC and 500ºC.

In an embodiment, the cartridges comprise an air-water exchanger in which cold or hot water is forced to pass through, where thermal exchanges with the environment air will be promoted.

In an embodiment, the cartridges comprise one fan that will force air circulation through the exchanger to alter temperature and condition air dispersion throughout space.

In an embodiment, the air quality enhancement exchanger and filtration for indoor confined or semi-confined spaces comprises a system that regulates the temperature of "purified" air, contributing to the thermal comfort of the users of confined or semi-confined spaces where the equipment acts.

In an embodiment, the air quality enhancement exchanger for indoor confined or semi-confined spaces comprises a system that dehumidifies the "purified" air, also contributing to the comfort of the users of confined or semi-confined spaces where the equipment acts.

The present disclosure also describes the method of functioning of an air quality enhancement exchanger and filtration for indoor confined or semi-confined spaces occupied by people or animals, which comprises the steps of:
- Injection of flow of air and/or gases through a controlled force action system in the air intake area;
- First step, passage through a water filtration system, composed by the storage water, with an integrated far-UVC or UVC action system protected of the direct contact water, by a non-isolation UVC radiation such us glass material or others equivalent, for the first microbial inactivation system filtered in water, because the microorganism filtered in water are in a confined space where the application of UVC are very efficiency, a decrease the physical particles move to the next system, composed by the UVC exchanger and the security of the maintenance and substitution of the water without the necessity of special equipment and/or an specialized human;
- Passage through the microbial inactivation system, applicable to viruses, bacteria and other microorganisms, through UV action, with a length of 207 to 254 nm, applied for far-UVC or UVC emissions system; and a sinuous circuit that guarantees an increase in the exposure time of the air and/or gas to UV radiation;
- Possibility of applying a cartridge that allows the passage through protective filters, composed by an activated carbon or similar filter action to improve odour quality and minimize the presence of little dimension particles, such as pollen, dust, dust mite, hair animals, hair people, among others with smaller-sized pores capable of filtering smaller and radioactive molecules not filtered in water filter in the first moment;
- Possibility of applying a cartridge that allows the passage through dehumidification system, contributing to the comfort of the users of confined or semi-confined spaces where the equipment acts;
- Possibility of applying a cartridge that allows the passage through an upgrade actuation system, comprising a heating or cooling system with a heat and/or cold existing or new systems of the space or the building for creating a contact temperature with the flow of air and/or gases before the insufflation in the space;
- Possibility of applying a cartridge that allows the passage through an upgrade actuation system, comprising a heating system with a heat exchanger for creating a contact temperature with the flow of air and/or gases of 70ºC to 500ºC, when the previous cartridge presented in vi) is not a solution because the space or building do not have a heat and or cold circuit system, contributing to the comfort of the users of confined and semi-confined spaces where the equipment acts;
- Possibility of applying a cartridge that allows the passage through dehumidification regulation system, contributing to the quality air;
- The solution has a control system, that integrated CO, CO₂ and O2 sensors, with a luminous, sonorous and or internet communications alarms, to control the quality of the air and generate and acquisition data for the users or the operator control system;
- The air and/ or gases outflows zone presented the quality and the comfort needed for the indoor confined and semi-confined spaces occupied by people or animals.

This present disclosure also describes the method of functioning of an air quality enhancement exchanger and filtration for indoor confined or semi-confined spaces occupied by people or animals, which considers the exposure times to UVC radiation, as different exposure times are needed to cause the inactivation of different microorganisms, protective filters, temperature control and dehumidification. This technology allows microbiological inactivation by increasing the exposure time and reducing the space occupied by the system, thus achieving the inactivation of microorganisms of different cadences. It also allows effective filtering of physical particles, not only microorganisms, but also others such us dust, pollen, dust mites, animal hair, human hair, textile and non-textile fibres, radioactivity molecules, such as radon, among others, which cause a loss of air quality and human, animal and public health problems. It also allows having a control system, sensors and alerts for indoor air quality, which emit light, sound and/or Bluetooth, Wi-Fi or SCard signals for IOT or mobile communications.

As a result, indoor air is improved, protecting people or animals who share these confined and semi-confined spaces. This technology can also easily incorporate a reduced dimensions equipment that allows the effective inactivation of microorganisms present in the air and/or gases in confined and semi-confined spaces, occupied by people or animals and at a relatively low cost. Its versatility and easy integration into different environments, such as rooms, buildings, among others, becomes a great advantage, as physical space is often a limiting factor.

This application also describes another operating mode of the air quality enhancement exchanger for indoor confined and semi-confined spaces occupied by people or animals, which is based on the Venturi Effect. It comprises a sinuous circuit designed to increase the time the air and/or gas stays inside the equipment and, consequently, the time of exposure to far UV-C radiation, with such length that allows the maximization of the exposure time of the percolated air to radiation to effectively inactivate any microorganism. The outlet of the air and/or gas occurs through a grille slightly shorter in length than the equipment, at its front. This outlet forms a surface that includes a constriction, allowing the homogeneous outlet of air and/or gas from the equipment.

An aspect of the present disclosure relates to an air exchanger device, for improving indoor air quality in confined and semi-confined spaces occupied by people or animals, comprising: at least one air intake area (4) for air and/or gas inflow; and at least one air exit area (5) for air and/or gas outflow; wherein the air and/or gas flows from the at least one air intake area (4) to the at least one air exit area (5) through a sinuous circuit comprising a winding section (6) placed inside said device; the air exchanger device further comprising, at least one UV-C radiation lamp (7), in particular, a far-UV-C radiation lamp, mounted inside the device along the sinuous circuit, for inactivating microorganisms present in the air or gas flow.

In an embodiment, the air exchanger device comprises a rapid detection module (8), in particular comprising at least one UV-C intensity sensor, configured for real-time monitoring of the at least one UV-C radiation lamp.

In an embodiment, the air exchanger device comprises at least one gas concentration sensor configured to detect concentration levels of gases, such as CO₂, O₂, and/or CO, for monitoring and optimizing air quality within the confined or semi-confined spaces.

In an embodiment, the air exchanger device comprises a set of audible alarms configured to emit sound alerts when the concentration levels of gases, such as CO₂, O₂, and/or CO, detected by the at least one gas concentration sensor exceeds predefined threshold values.

In an embodiment, the air exchanger device comprises a wireless communication module configured to transmit alerts generated by the at least one gas concentration sensor directly to a user's device, in particular via Bluetooth and/or Wi-Fi, when the concentration levels of gases, such as CO₂, O₂, and/or CO, detected by the at least one gas concentration sensor exceed predefined threshold values.

In an embodiment, the air exchanger device comprises a control unit configured to monitor and receive data from the rapid detection module (8) and/or the at least one gas concentration sensor; and further configured to regulate airflow through the intake area (4) and exit area to (5), in particular intake and exit flow rates, for improved air treatment efficiency.

In an embodiment, the air exchanger device comprises a display configured to show data generated by the control unit related to the indoor air quality.

In an embodiment, the air exchanger device comprises comprising one or more cartridges (9), wherein each of the one or more cartridges have an air and/or gas inlet and an air and/or gas outlet and wherein each of the one or more cartridges are configured as: an activated carbon filter (11) for removing volatile organic compounds and odours from the air and/or gas, or a radon filter (12) for absorbing radon gas particles and reduce their concentration in indoor air and/or gas , or a water filter for trapping particulate matter, impurities, and gases through air-water interaction to purify and humidify the air and/or gas, or a user-controlled heating system (13) for regulating the temperature of the air and/or gas exiting the air exchanger device.

In an embodiment, the user-controlled heating system (13) comprises a heat source configured to generate temperatures from 70ºC to 500ºC, for regulating the temperature of the air and/or gas exiting the air exchanger device.

In an embodiment, the one or more cartridges (9) are detachably mounted between the air intake area (4) and the sinuous circuit (6).

In an embodiment, at least one further UV-C radiation lamp (14) is housed at a bottom compartment of the one or more detachably mounted cartridges (9).

In an embodiment, the at least one UV-C radiation lamp (7, 14) operates at wavelengths between 190 nm and 250 nm and/or between 253 nm and 255 nm.

In an embodiment, a cross-sectional area of the sinuous circuit (6) for air and/or gas flow decreases progressively from the air intake area to the air exit area, for accelerating the flow of air and/or gas therein.

In an embodiment, the at least one air intake area (4) is placed in an upper part of the exchanger device for intake of air and/or gas from an upper area of an indoor space; and the at least one air exit area (5) is placed in a lower part of the exchanger device for the outflow of air and/or gas to a lower area of an indoor space.

A further aspect of the present disclosure relates to an air exchanger system comprising the air quality exchanger wherein the at least one air intake area (4) is connected to a controlled forced air inflow device; and/or the at least one air exit area (5) is connected to a controlled forced air outflow device.

In an embodiment the at least one air intake area and/or the at least one air exit are connected to at least one flow regulator valve, and/or cut-off valves, and/or mechanical and electrical actuation devices.

In an embodiment the air exchanger system comprises an air-water exchanger in which cold or hot water is forced to pass through.

In an embodiment the air exchanger system comprises one fan configured to circulate air and/or gas through the air exchanger device.

Another aspect of the present disclosure relates to a method of operating the air exchanger device for improving indoor air quality in confined or semi-confined spaces, the method comprising: drawing air and/or gas through the at least one air intake area (4); directing the air and/or gas through the sinuous circuit (6) within the air exchanger device; exposing the air and/or gas to the at least one UV-C radiation lamp (7, 14) positioned along the sinuous circuit, to inactivate microorganisms present in the air or gas flow; and releasing the treated air and/or gas through the at least one air exit area (5).

A further aspect of the present disclosure relates to the use of the air exchanger device in building ventilation systems for improving indoor air quality.

A further aspect of the present disclosure relates to the use transportation systems for improving indoor air quality.

The term "comprises" or "comprising" when used in this document is intended to indicate the presence of the characteristics, elements, integers, steps and components mentioned, but does not preclude the presence or addition of one or more other characteristics, elements, integers, steps and components, or groups thereof.

The present disclosure is in no way restricted to the realizations described in this document and a person with average knowledge of the field will be able to foresee many possibilities for modifying it and replacing technical characteristics with equivalent ones, depending on the requirements of each situation, as defined in the appended claims.

This present disclosure addresses a system for improving indoor air quality enhancement exchanger in shared spaces.

The system that has been developed is slanted towards providing protection for contact and interaction between people and indoor air in buildings and other services that might give rise to situations involving contact at less than two meters between two or more people.

It was developed on the basis of fluid mechanics, allowing possible contacts with airborne viruses, bacteria and other microorganisms to be steered into an inactivation, decontamination and sterilization zone, with the combined and modular integration of precise and directed UV radiation emissions, air physical filtrations, thermal comfort and dehumidification modules, with capacity to generate upgrades of the system, and individual, easy, security, and low cost maintenance.

The system that has been developed is intended to ensure the improvement of the air quality in indoor confined or semi-confined spaces occupied by people or animals, by the creation of a system that may or may not be coupled to another air renewal and climatization systems previously installed in the specific space. It involves the combination of the interaction between the fluid mechanics of the system and the part emitting UV radiation at wavelengths between 190 nm to 250 nm and/or 253 nm to 255 nm, avoiding turbulent regimes and dead zones with a higher concentration of virus, bacteria, and other microorganisms.

This is a system and technology for immediate application, responding to immediate needs and providing medium and long-term protection for healthcare practitioners, patients, and other users of healthcare facilities, as well as in any other confined and semi-confined spaces occupied by people or animals with significant impact on improving public health.

This is a system and technology that allows the joint or separate application of cartridges with multiple functions, depending on the intended use of the equipment, to ensure the safety and well-being of individuals in confined and semi-confined spaces occupied by people or animals.

### BRIEF DESCRIPTION OF THE DRAWINGS

The following figures provide preferred embodiments for illustrating the disclosure and should not be seen as limiting the scope of the present disclosure.

For an easier understanding of this application, the Figures are appended hereto, presenting embodiments that nevertheless make no attempt to limit the technique disclosed herein.
Fig. 1 - illustrates the schematic representation of an embodiment of the present disclosure, which shows at least one air intake area (4), that allows air inflow (1), at least one air exit area (5), that allows air outflow (2), the sinuous circuit of the UV-C exposure zone circuit (6), the far-UV-C radiation lamps (7) and the rapid detection module (8), the casing (24) and the fan (3).
Fig. 2 - illustrates the side view of the air path along the rapid detection module for supervision UV-C module (8), semi-confined spaces (9), including the reflective internal coating (22), and small compartment at the bottom of the cartridge (10).
Fig. 3 - illustrates the cross-section of the water filtration stage (25) - including the air-water contact zone (13), the reservoir (14) and the dispersion medium (15) - , carbon filter (11), radon filter (12), user-controlled heating system (13), with a UV-C lamp source (14), and a small compartment at the bottom of the cartridge (10) to ensure the maintenance of this modules.
Fig. 4 - illustrates the enlarged detail of plug in System (15) of each additional module , illustrating the capacity and friendly user maintenance system.
Fig. 5 - illustrates a section of Venturi effect zone and the sinuous circuit (16).
Fig. 6 - illustrates the flow regimes, showing laminar flow in the UV-C zone (16) and controlled turbulence (17) through diffusers (20, 18), with the UV safety sensor (21) and flow/pressure sensor (23), and the control unit (19).

Along the figures, the following items are appointed by the following reference numbers:
- 1 -: Air inflow;
- 2 -: Air outflow;
- 3 -: Fan/air circulation medium;
- 4 -: Air intake area;
- 5 -: Air exit area;
- 6 -: Sinuous circuit of the UV-C exposure zone;
- 7 -: UV-C radiation lamps/module;
- 8 -: Rapid detection module for supervision UV-C module;
- 9 -: Semi-confined spaces;
- 10 -: Small compartment at the bottom of the cartridge;
- 11 -: Carbon Filter;
- 12 -: Radon Filter;
- 13 -: User-controlled heating system;
- 14 -: UVC emission lamps;
- 15 -: Plug in System;
- 16 -: Venturi effect zone: sinuous circuit;
- 17 -: air intake;
- 18 -: air outlet;
- 19 -: control system;
- 20 -: Grille;
- 21 -: UV safety sensor;
- 22 -: reflective internal coating;
- 23 -: Flow/pressure sensor;
- 24 -: Cover or maintenance access;
- 25 -: Water filtration stage;
- 26 -: Air-water contact zone;
- 27 -: Water reservoir;
- 28 -: water dispersion medium;
- 29 -: device casing.

### DETAILED DESCRIPTION

The present disclosure relates to an air quality improvement device.

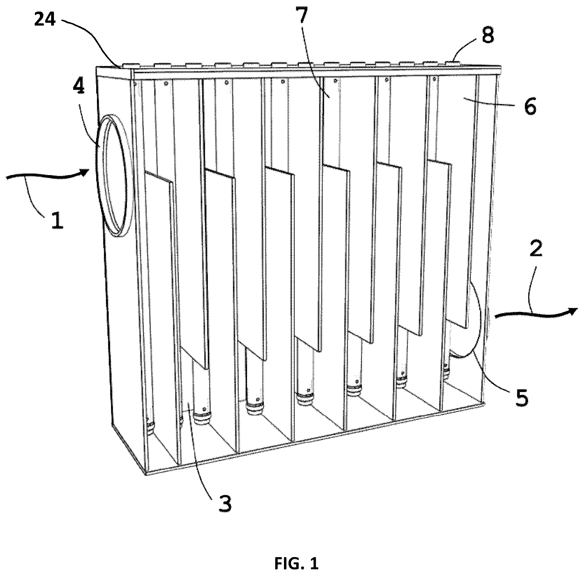

The general concept of the present disclosure is depicted in Fig. 1, which illustrates a device designed to protect people or animals against microorganisms suspended in the air, such as viruses, bacteria, and fungi. In this system, air and/or gases are drawn into a closed treatment device and directed through an internal air flow circuit comprising a winding section (6) forming a sinuous path, in which the air is subjected to inactivation, decontamination, and sterilisation by UV-C radiation, without adversely affecting the health or safety of occupants.

In an embodiment, the sinuous circuit (6) comprises a plurality of sheets. Preferably, a first subset of the plurality of sheets has a first end attached to a bottom portion of the device, and a second subset of the plurality of sheets has a first end attached to a top portion of the device. Each sheet thus has a first end fixed to the device and a second end, opposite the first end, which is free.

Preferably, the sheets are arranged alternately such that a sheet having a first end attached to the bottom portion of the device is followed by a sheet having a first end attached to the top portion of the device, and so on.

Preferably, each sheet has a length shorter than the distance between the bottom and top portion of the device, such that the free end of each sheet is spaced apart from the corresponding top portion or bottom portion of the device by a predefined distance, thereby defining a passage for airflow.

In an embodiment, the sheets are interleaved and arranged so as to define a meandering airflow path, the sheets being preferably offset relative to one another along the direction extending from the air intake to the air exit, thereby increasing the effective flow path length and the residence time of the air within the device.

Initially, and depending on the intended application, interaction context, and characteristics of the indoor space, the minimum air intake and exhaust conditions are defined through the air inlet (1) and the treated air outlet (2). These conditions are selected to ensure effective air circulation, formation of air curtains where applicable, and conveyance of airborne microorganisms toward the treatment device. The determination of these conditions may be supported by numerical simulations, such as finite element analysis or computational fluid dynamics models.

The selection and positioning of the air inlet (14) and treated air outlet (2), as illustrated in Fig. 1, depend on several factors, including the geometry and aerodynamic characteristics of the space, the presence of physical obstacles, expected occupancy levels, room dimensions, and the available installation locations for air circulation components, with the objective of directing contaminated air toward the internal treatment zones of the device.

Based on the defined design parameters, a control unit (19) regulates the operation of the fan or air circulation medium (3) so as to balance the volumetric air inflow and outflow. Maintaining substantially equal inflow and outflow promotes stable airflow conditions with minimal turbulence, particularly within the UV-C exposure zone (11), thereby enabling predictable residence times and uniform treatment.

The free cross-sectional area of the internal air flow circuit (4), including the winding section (6), is selected to be equal to or smaller than the effective cross-sectional area of the air inlet (4), ensuring controlled airflow velocity and preventing bypass of untreated air.

The airflow through the device is configured to operate under substantially steady-state conditions, such that the volumetric flow entering through the air inlet (4) corresponds to the volumetric flow exiting through the treated air outlet (5, 18). This principle applies to applications in buildings and in transportation environments, including airborne, terrestrial, and maritime systems.

In an embodiment, as illustrated in Fig. 1, the internal air flow circuit (4) comprises a winding section (6) defining a sinuous path and a UV-C radiation module (7), which emits radiation in a wavelength range from 100 nm to 280 nm, enabling the inactivation of viruses, bacteria, and other microorganisms. In preferred embodiments, the UV-C radiation sources (7, 14) operate in the wavelength range from 190 nm to 250 nm and/or from 253 nm to 255 nm, depending on the selected emission technology. The dimensions of the winding section (6) are selected to allow the treated air volume to pass through the UV-C exposure zone (7) under controlled flow conditions. The effective geometry of the circuit is defined in relation to the airflow velocity generated by the fan or air circulation medium (3). Control of airflow velocity within the winding section (6) ensures sufficient exposure time for microbiological inactivation, with embodiments achieving particle inactivity rates of at least 99.9%. Flow or pressure sensors (23) may be provided to monitor airflow conditions within this section.

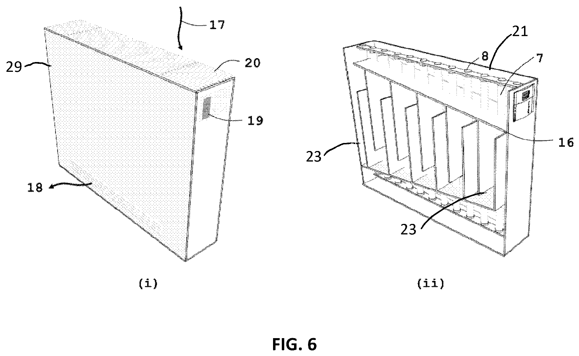

In an embodiment, also shown in Fig. 1, the UV-C radiation source/module (7) may be associated with a UV safety sensor (21), as illustrated in Figure 6, configured to monitor the operational status of the UV-C radiation sources (7, 14), with a visual, quick and easy access and supervision (8) and complemented in (10), enabling detection of lamp degradation, malfunction, or replacement requirements.

To increase system flexibility, modular treatment stages may be integrated within the device casing (29), as illustrated in Fig. 6. These stages may be added or removed through a cover or maintenance access (24), allowing the system to be adapted to different indoor air quality requirements.

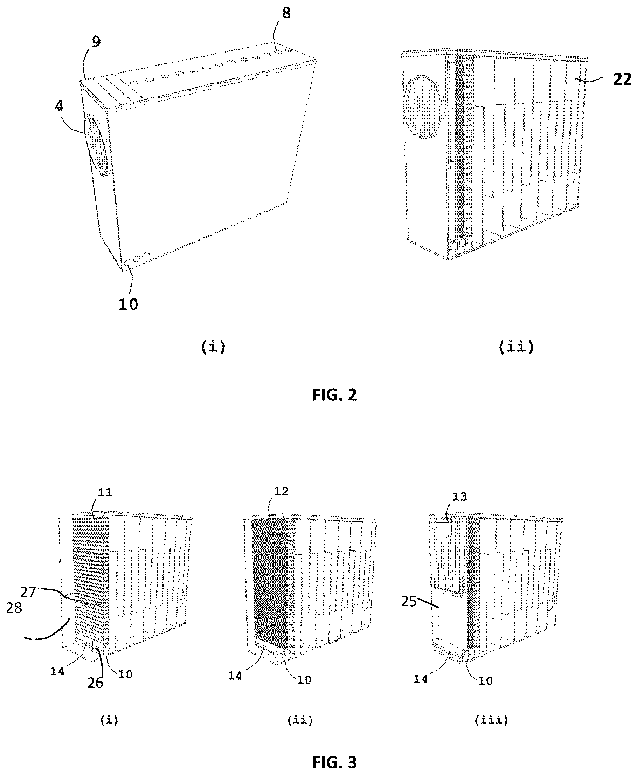

In certain embodiments, the internal air flow circuit (4) includes a water filtration stage (25), as illustrated in Fig. 3, comprising an air-water contact zone (13), a water reservoir (27), and a water dispersion medium (28). This stage enhances removal of particulate matter, odours, and soluble contaminants through humid air washing.

In addition, or alternatively, a radon reduction module (12) may be integrated, comprising a radon adsorbent medium configured to remove radioactive gases such as radon from the airflow. This improves system effectiveness in environments where radon contamination is present.

In some embodiments, a thermal treatment stage may be incorporated to raise the temperature of the airflow to levels exceeding 70°C and up to approximately 500°C, enhancing inactivation of microorganisms when UV-C treatment alone is insufficient due to space or flow constraints.

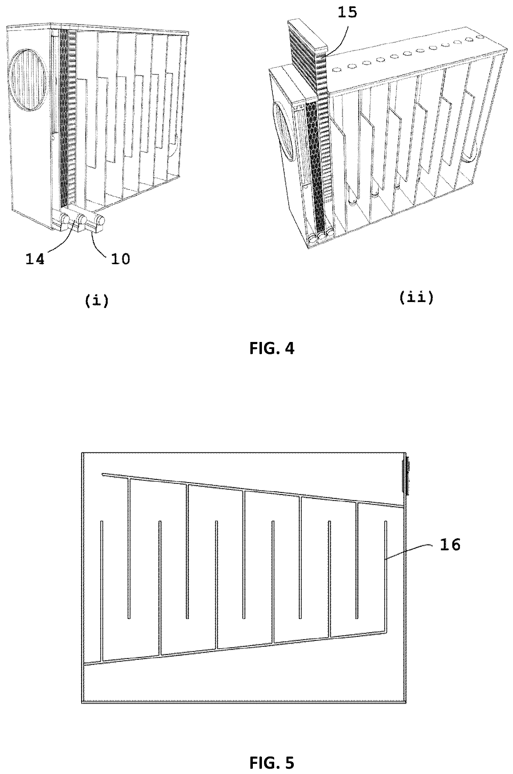

Figure 4 illustrates an embodiment in which the UV-C radiation sources (14) are removably mounted on corresponding UV-C (10) additional and independent sources , allowing safe and convenient replacement through the maintenance access (24 - represented in Figure 1). The reflective internal coating (22) enhances irradiation efficiency within the UV-C exposure zone (6), originated by UV-C Lamps (7) and with the same impact for UV-C radiation sources (14).

Figure 5 illustrates an alternative embodiment in which the geometry of the internal air flow circuit (4) is adapted to generate a Venturi effect, producing controlled acceleration of airflow followed by velocity reduction within the winding section (6). This configuration (16) increases residence time within the UV-C exposure zone and improves microbiological inactivation efficiency.

The Venturi-based constriction relies on controlled reduction of flow cross-section, resulting in pressure variation and airflow homogenisation, thereby promoting uniform treatment of air throughout the device.

Figure 6 illustrates a further embodiment in which the air inlet (1) and treated air outlet (18) are arranged in an upper-lower configuration. Controlled turbulence zones (7) and diffuser or mixing grids (20) are positioned upstream and/or downstream of the UV-C exposure zone (8) to promote homogeneous airflow distribution. The control unit (19) and flow or pressure sensors (23) are also shown.

The internal view of Fig. 6 further illustrates the UV-C radiation module (7) and associated UV safety sensors (21), enabling continuous verification of proper radiation emission.

The device is dimensioned to ensure delivery of a minimum effective UV-C dose to airborne microorganisms, achieved through a controlled relationship between irradiance, airflow velocity, and residence time within the internal air flow circuit (4).

The internal aerodynamics are engineered to maintain predominantly laminar flow within the UV-C exposure zone (6), while controlled turbulence is introduced in the turbulence zones (7) to eliminate dead zones and promote homogeneous mixing.

The UV-C radiation sources (7, 14) are positioned substantially parallel to the airflow direction, minimising shadowing and ensuring uniform irradiance distribution. The reflective internal coating (22) further enhances dose efficiency.

In embodiments including water filtration, the water filtration stage (10) is dimensioned based on air-water contact time, interfacial area, and reservoir volume to ensure effective washing and stable operation.

In embodiments including radon treatment, the radon reduction module (16) is preferably positioned downstream of the UV-C exposure zone (6) and water filtration stage (25), preventing contamination of the radon adsorbent medium (12).

Controlled turbulence devices such as mixing grids (20) are positioned outside the UV-C exposure zone (6) to avoid dose reduction while ensuring homogeneous airflow.

In an embodiment, the device casing is opaque to UV-C radiation and incorporates safety features including UV safety sensors (21), access monitoring at the cover or maintenance access (24), and operational monitoring by the control unit (19).

The modular architecture allows deployment in residential, educational, healthcare, office, and large shared indoor environments.

In an embodiment, the device is scalable according to room volume, occupancy, and airflow requirements.

Functional parameter ranges governing UV-C dose, airflow velocity, circuit geometry, surface reflectance, water filtration, and radon reduction are defined as preferred but non-limiting design guidelines.

The technology may be implemented using equivalent values or configurations achieving the same technical effects of microbiological reduction, indoor air quality improvement, and safe enclosed UV-C treatment.

Referring to the Figures, some embodiments are now described in greater detail, although they are not intended to limit the scope of the present application.

The disclosed device is designed taking into account a set of critical physical and operational parameters that govern the effectiveness, safety, and scalability of UV-C-based air treatment. In particular, the device is dimensioned to ensure the delivery of a minimum effective UV-C dose to airborne microorganisms, expressed in millijoules per square centimetre, which is achieved through a controlled relationship between UV-C irradiance, air velocity, and residence time within a sinuous internal circuit. The airflow velocity, the effective length and geometry of the sinuous circuit, the exact positioning and power of the UV-C radiation sources, and the internal surface reflectivity are jointly selected so that the cumulative dose received by the air exceeds a minimum effective threshold for microbiological inactivation, while avoiding excessive energy consumption or unsafe radiation leakage.

The internal aerodynamics of the device were carefully engineered to balance laminar and turbulent flow regimes. In the UV-C exposure zone, the airflow is maintained in a predominantly laminar or quasi-laminar regime, thereby ensuring uniform irradiance distribution and preventing local under-dosing caused by flow short-circuiting. Upstream and downstream of the UV-C exposure zone, controlled turbulence is intentionally introduced to promote homogeneous mixing of the air mass, eliminate dead zones, and equalise the microbiological load prior to treatment and before reinjection into the indoor space. The design of bends, transitions, and curvature radii within the sinuous circuit avoids sharp angles and excessive pressure losses, favouring smooth flow paths that preserve predictable residence times.

The effective UV-C dose delivered within the device is determined by the average irradiance in the exposure section and the time during which the air remains within the irradiated volume. The residence time is a function of the effective path length of the sinuous circuit and the mean air velocity through that section. The device therefore relies on dose accumulation rather than peak power alone, allowing the same level of microbiological inactivation to be achieved either by increasing the effective path length, reducing airflow velocity within the UV-C zone, increasing the useful radiant power of the UV-C sources, or improving the internal reflectance of the circuit walls. For an embodiment, the device can be defined as delivering a minimum effective UV-C dose greater than 10 mJ/cm², without restriction to specific pathogens, while preferred embodiments are dimensioned to provide higher engineering safety margins.

The UV-C radiation sources are positioned along the sinuous circuit in a configuration substantially parallel to the airflow direction, rather than facing the flow directly. This arrangement minimises shadowing effects, improves irradiance uniformity, and reduces the risk of localised overexposure of internal materials. The lamps or emitters are distributed symmetrically along the exposure path, with spacing and distance to the airflow selected so that overlapping irradiance fields produce a uniform dose profile, with acceptable variation limits. The internal surfaces of the circuit are coated or manufactured from materials exhibiting high reflectance in the UV-C wavelength range, such as polished or anodised metals or technical polymer coatings, thereby increasing the effective irradiance through diffuse and/or specular reflection and reducing the required lamp power for a given dose.

In addition to dry UV-C treatment, the device optionally incorporates a wet air-washing stage based on controlled air-water interaction. This filtration by water stage is configured to capture particulate matter, bioaerosols, and soluble gaseous fractions through physical contact with a liquid medium, while also enabling controlled humidification. The effectiveness of this stage depends on the air-water contact time, the specific interfacial area, the volume of the water reservoir, and the recirculation regime. The water filtration module is dimensioned to provide sufficient contact time for effective capture while maintaining stable operation and manageable maintenance requirements. Where required, the water volume and circulation are selected in relation to the device airflow rate to ensure consistent performance across different installation scales.

In an embodiment, the device further includes a dedicated radon reduction module, recognising that radon is not inactivated or removed by UV-C radiation. The radon reduction function is therefore achieved using physical removal mechanisms, preferably based on adsorption technologies such as activated carbon, impregnated carbon, specialised zeolites, or hybrid adsorption media, optionally combined with controlled dilution or exhaust strategies. The radon reduction module is dimensioned according to the device airflow rate and acceptable pressure losses and is preferably positioned downstream of the UV-C and water filtration stages in order to prevent contamination or premature saturation of the adsorbent media. Passive or active radon monitoring may be integrated to assess performance and support maintenance decisions.

Specific design provisions are implemented in regions where turbulence is intentionally introduced. These provisions may include static mixing elements, low-pressure-drop diffusers, or flow-conditioning grids, positioned upstream and downstream of the UV-C exposure zone but never within the direct radiation field. The purpose of these regions is to homogenise the microbial and particulate concentration of the airflow, prevent stratification, and ensure that all treated air has been subjected to substantially equivalent treatment conditions.

In an embodiment, the entire device is enclosed within a structure that is opaque to UV-C radiation and manufactured from materials resistant to prolonged UV exposure. Safety features include radiation containment, interlocks or sensors associated with access panels or maintenance openings, continuous monitoring of UV-C source status, airflow, pressure losses, and, where applicable, ozone concentration. In embodiments intended for operation in shared environments such as campuses, common areas, meeting rooms, or indoor spaces with particulate or dust presence, additional monitoring of ozone levels may be provided to ensure compliance with indoor air quality standards and to verify that the UV-C sources operate within safe emission limits.

The modular architecture of the device allows deployment in residential environments, educational facilities, healthcare and care institutions outside critical zones, office buildings, and large shared indoor areas. The device is scalable and configurable according to room volume, occupancy, and air exchange requirements, enabling adaptation to different operational contexts while maintaining consistent microbiological reduction performance and indoor air quality improvement without direct exposure of occupants to ultraviolet radiation.

The embodiments of the device are dimensioned and configured on the basis of functional parameter ranges that ensure effective microbiological reduction, stable aerodynamic behaviour, and safe operation, while allowing scalability to different airflow rates and installation contexts. The following parameters represent preferred but non-limiting intervals for design and operation.

With respect to UV-C irradiation and airflow, in an embodiment of the device, which is configured to deliver an effective UV-C dose, designated D, within a functional range from 10 to 50 mJ/cm², preferably from 20 to 40 mJ/cm². More preferably, the device is dimensioned to deliver an effective dose equal to or greater than 30 mJ/cm², providing a robust engineering margin for generic microbiological reduction without reference to specific pathogens. In an embodiment, the airflow velocity v within the UV-C exposure section is selected within a functional range from 0.2 to 2.0 m/s, with preferred operation from 0.2 to 1.0 m/s in order to increase residence time and improve irradiance uniformity. In an embodiment, the effective path length Lₑf of the sinuous circuit is typically selected within a range from 1.0 to 6.0 metres, preferably from 1.5 to 4.0 metres, allowing residence time to be increased without a proportional increase in system volume. The distance d between the UV-C radiation sources and the main airflow is selected within a functional range from 30 to 200 mm, with preferred distances from 50 to 150 mm, in order to optimise irradiance distribution. In an embodiment, the device is designed such that irradiance uniformity along the exposure section is maintained within a variation of approximately ±20%, thereby avoiding localised under-dosing.

The internal surfaces of the UV-C exposure circuit are selected and treated to provide high reflectance in the UV-C wavelength range. The UV-C reflectance coefficient ρ of the internal lining is selected within a functional range from 0.70 to 0.95, with preferred values equal to or greater than 0.85 in order to increase effective irradiance through multiple reflections. The internal surface roughness and finish are controlled and maintained at low levels to minimise aerodynamic losses, particle deposition, and degradation of reflective performance over time.

In embodiments incorporating air-water filtration, the device comprises a wet contact stage dimensioned according to critical hydraulic and mass-transfer parameters. The air-water contact time t₍w₎ is selected within a functional range from 0.3 to 2.0 seconds, with preferred values from 0.5 to 1.5 seconds to ensure effective capture of particles, bioaerosols, and soluble gaseous fractions. The water reservoir volume V(w) is selected within a functional range from 1 to 20 litres, with preferred values corresponding from 2 to 10 litres per 100 m³/h of treated airflow, providing operational stability and buffering capacity. The specific interfacial contact area a between air and water is selected to be at least 200 m²/m³, preferably equal to or greater than 300 m²/m³, in order to maximise washing efficiency without excessive pressure losses.

For radon reduction, the device integrates a dedicated module employing physical removal mechanisms, recognising that radon is not affected by UV-C radiation. Applicable technologies include adsorption-based solutions using activated carbon, impregnated carbon, specialised zeolites, hybrid adsorption media, and combinations thereof, optionally supplemented by controlled dilution or exhaust strategies. Adsorption-based solutions using activated carbon and/or zeolites are preferred due to their compatibility with compact designs and predictable performance. The radon reduction module is positioned within the circuit either before or after UV-C and water filtration stages, with a preferred placement downstream of both UV-C irradiation and air-water filtration in order to avoid contamination or premature saturation of the adsorbent medium.

From an aerodynamic perspective, the airflow regime within the UV-C exposure section is controlled to maintain laminar or quasi-laminar conditions. The Reynolds number Re in the UV-C section is maintained below approximately 4000, with preferred values below 2300, ensuring flow stability and uniform dose distribution. Controlled mixing devices such as flow grids, static helicoidal mixers, or low-pressure-drop diffusers may be incorporated upstream and/or downstream of the UV-C exposure zone. These devices are positioned outside the direct radiation field and are configured to homogenise airflow and microbiological concentration without reducing the effective UV-C dose delivered within the exposure section.

The above parameter ranges are provided as functional and preferred design guidelines and do not limit the scope of the technology, which may be implemented using equivalent values or configurations that achieve the same technical effects of effective microbiological reduction, air quality improvement, and safe enclosed UV-C treatment.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. An air exchanger device, for improving indoor air quality in confined and semi-confined spaces, comprising:
at least one air intake area (4) for air and/or gas inflow;
at least one air exit area (5) for air and/or gas outflow;
an internal air flow circuit (6) extending from the air intake area to the air exit area, the circuit comprising a plurality of sheets offset from one another along a direction extending from the air intake to the air exit to define a sinuous airflow path;
at least one UV-C radiation source (7, 14) for emitting a UV-C radiation, arranged in the airflow circuit along the sinuous airflow path as to define a UV-C exposure zone for inactivating microorganisms present in the air or gas flow;
wherein the sinuous airflow path comprises an effective length and cross-section selected such that, for a predetermined airflow rate, a residence time of the air within the UV-C exposure zone (6), in combination with an average UV-C irradiance provided by the at least one UV-C radiation source, results in a minimum effective UV-C dose greater than 10 mJ/cm² to the air flowing through the circuit; and
wherein the plurality of sheets comprise a UV-reflective coating (10) having a UV-C reflectance coefficient of at least 0.70.

2. The device according to the previous claim, wherein airflow within the UV-C exposure zone (6) is maintained in a laminar or quasi-laminar regime with a Reynolds number below 4000, preferably below 2300.

3. The device according to any of the previous claims, wherein the at least one UV-C radiation source (7) is arranged substantially parallel to the plurality of sheets.

4. The device according to any of the previous claims, wherein the UV-C reflective coating (22) comprises a reflectance coefficient of at least 0.85.

5. The air exchanger device according to the previous claim further comprising a rapid detection module (8), in particular comprising at least one UV-C intensity sensor, configured for real-time monitoring of the at least one UV-C radiation source.

6. The device according to any of the previous claims, wherein the internal air flow circuit (4) is configured to receive one or more modular treatment stages, each module treatment stage being arranged downstream or upstream of the UV-C exposure zone (6).

7. The device according to any of the previous claims, wherein at least one modular treatment stage comprises a water filtration device (25) defining an air-water contact zone (26), configured to reduce particulate and bioaerosol load prior to UV-C exposure.

8. The device according to any of the previous claims, wherein the air-water contact zone (26) is dimensioned to provide an air-water contact time from 0.3 to 2.0 seconds and an interfacial area of at least 200 m²/m³.

9. The device according to any of the previous claims, wherein at least one modular treatment stage comprises a radon reduction module (12) comprising a radon adsorbent medium, positioned downstream of the UV-C exposure zone (6) so as not to interfere with UV-C irradiation or residence time within the exposure zone.

10. The device according to the previous claim, wherein the radon adsorbent medium comprises activated carbon, impregnated carbon, zeolites, or combinations thereof.

11. The device according to any of the previous claims, comprising at least one controlled turbulence zone (18) arranged upstream and/or downstream of the UV-C exposure zone (6), the controlled turbulence zone comprising one or more diffuser or mixing grids (20), static flow conditioners, or equivalent low-pressure-drop mixing elements.

12. The device according to any of the previous claims, further comprising at least one flow or pressure sensor (23) and/or UV safety sensor (21), configured to monitor operating conditions relevant to maintaining the minimum effective UV-C dose within the exposure zone (6), including airflow rate, pressure loss, and UV-C source status.

13. A method of operating an air exchanger device described in any of the previous claims, for improving indoor air quality in confined or semi-confined spaces, the method comprising:
drawing air and/or gas through the at least one air intake area (4);
directing the air and/or gas through the sinuous circuit (6) within the air exchanger device;
exposing the air and/or gas to the at least one UV-C radiation source (7, 14) positioned along the sinuous circuit, to inactivate microorganisms present in the air or gas flow; and
releasing the treated air and/or gas through the at least one air exit area (5).

14. Use of the air exchanger device described in any of the previous claims 1 to 12 in building ventilation systems for improving indoor air quality.

15. Use of the air exchanger device described in any of the previous claims 1 to 12 in transportation systems for improving indoor air quality.
